# EUROPÄISCHE PATENTSCHRIFT

(11) **EP 3 793 630 B1**
(45) Veröffentlichungstag und Bekanntmachung des Hinweises auf die Patenterteilung: **06.04.2022**
(21) Anmeldenummer: 19725346.1
(22) Anmeldetag: 16.05.2019
(51) Int. Cl.: F16C 17/10, F04D 29/048, F16C 17/26

(54) **ROTORLAGERUNGSSYSTEM**
ROTOR BEARING ARRANGEMENT
SYSTÈME DE PALIER DE ROTOR

(30) Priorität: 16.05.2018 DE 102018207611
(43) Veröffentlichungstag der Anmeldung: 24.03.2021
(73) Patentinhaber: Kardion GmbH, 70376 Stuttgart (DE)
(72) Erfinder: VOGT, Andreas, 71272 Renningen (DE); STOTZ, Ingo, 71254 Ditzingen (DE); BETTE, Johannes, 71229 Leonberg (DE); SCHUELKE, Armin, 71134 Aidlingen (DE); LI, Xiang, 70376 Stuttgart (DE); VOLLMER, Uwe, 71069 Sindelfingen (DE); MINZENMAY, David, 70569 Stuttgart (DE)
(74) Vertreter: Gauss, Nikolai
(86) Internationale Anmeldenummer: PCT/EP2019/062746
(87) Internationale Veröffentlichungsnummer: WO 2019/219883

(56) Entgegenhaltungen:
- DE-A1- 19 910 872
- DE-T2- 60 119 592

## Beschreibung

Die vorliegende Erfindung betrifft eine Pumpe für das Fördern eines Blutstroms zur Herz-Kreislauf-Unterstützung mit einem Rotor mit einer Rotationsachse zur Förderung einer Flüssigkeit, wobei der Rotor einen zweiten hohlzylinderförmigen Permanentmagneten aufweist, welcher um eine Drehachse drehbar gelagert ist; und mit einem Gehäuse, in dem ein erster Permanentmagnet zur Übertragung eines Drehmoments auf den Rotor um die Drehachse drehbar gelagert ist, wobei der erste Permanentmagnet und der zweite Permanentmagnet sich zumindest teilweise axial überlappen, und wobei sich in dem axialen Überlappungsbereich des ersten Permanentmagneten und des zweiten Permanentmagneten das Gehäuse zwischen den beiden Permanentmagneten befindet, mit einem ersten Lager zur relativen Positionierung des Rotors und des Gehäuses zueinander und mit einem zweiten Lager und einem dritten Lager zur Aufnahme von Radialkräften und zur Positionierung der Rotationsachse des zweiten Permanentmagneten.

Eine derartige Pumpe für das Fördern eines Blutstroms zur Herz-Kreislauf-Unterstützung ist aus der DE 601 19 592 T2 bekannt.

Die DE 199 10 872 A1 beschreibt eine Lageranordnung für Drehvorrichtungen mit Magnetlagern, die für das kontaktfreie Lagern eines Rotors in einem Stator dienen.

Im Stand der Technik sind Pumpen mit einer Kombination aus einem Festkörperlager und einer passiven magnetischen Axialkupplung bekannt. Ferner ist auch eine radiale passive magnetische Kupplung zur Drehmomentübertragung mit axialem Versatz eines Kupplungsteils, d. h. einer Vorspannung, zur Einstellung der Axialkraft bekannt.

Die Verwendung einer axialen passiven Magnetkupplung führt zu einer hohen Axialkraft auf die Lagerung und somit zu erhöhter Reibung und erhöhtem Verschleiß. Hierbei ist eine Minimierung der vom Lager aufzunehmenden Axialkraft nicht möglich, da diese direkt abhängig vom zu übertragenden Moment ist.

Darüber hinaus ist auch eine Anordnung mit zwei Festlagern zur axialen und radialen Rotorlagerung in Pumpen, insbesondere bei Pumpen zur Herz-Kreislauf-Unterstützung (VAD), bekannt. Bei vorgespannten Lagern stellt die Verwendung zweier Festkörperlager eine überbestimmte Lagerung dar, bei der durch Verschleiß des belasteten Lagers oder der belasteten Lager die Vorspannung solange reduziert wird bis sich ein kleines Spiel einstellt und die Lagerung unterbestimmt wird, was nachteilhaft ist. Weiterhin kann eine thermisch bedingte Längung bzw. Ausdehnung des Rotors nicht kompensiert werden, so dass es zum Verklemmen des Rotors zwischen den beiden Axiallagern kommen kann.

Aufgabe der Erfindung ist es, ein Rotorlagerungssystem bereitzustellen, in dem geringere Reibung und damit weniger Verschleiß auftritt.

Diese Aufgabe wird durch das in Anspruch 1 angegebene Pumpe für das Fördern eines Blutstroms zur Herz-Kreislauf-Unterstützung gelöst. Vorteilhafte Ausführungsformen der Erfindung sind in den abhängigen Ansprüchen angegeben.

In einer erfindungsgemäßen Pumpe kann ein Drehmoment auf einen sich drehenden Rotor berührungsfrei übertragen werden, der in axialer und radialer Richtung gelagert ist.

Eine erfindungsgemäße Pumpe weist ein Gehäuse auf, in dem ein erster insbesondere zylinderförmiger Permanentmagnet um eine erste Achse drehbar gelagert ist.

Ferner weist eine erfindungsgemäße Pumpe einen Rotor auf, welcher einen zweiten hohlzylinderförmigen Permanentmagneten aufweist, welcher um eine zweite Achse drehbar gelagert ist. Bevorzugt weist der Rotor einen hohlzylinderförmigen Teil auf, in dem der zweite hohlzylinderförmige Permanentmagnet angeordnet ist.

Bei einer erfindungsgemäßen Pumpe überlappen der erste Permanentmagnet und der zweite Permanentmagnet axial zumindest teilweise, wobei der erste Permanentmagnet gegenüber dem zweiten Permanentmagneten axial versetzt angeordnet.

Unter dem Ausdruck, dass der erste Permanentmagnet gegenüber dem zweiten Permanentmagneten axial versetzt angeordnet ist, wird verstanden, dass ein axialer Mittelpunkt des ersten Permanentmagneten gegenüber einem axialen Mittelpunkt des zweiten Permanentmagneten axial versetzt angeordnet ist.

Hierbei berechnet sich der axiale Mittelpunkt eines Permanentmagneten als Punkt zwischen dem einen axialen Ende des Permanentmagneten und dem entgegengesetzten anderen axialen Ende des Permanentmagneten. Hierbei liegt ein axiales Ende auf einer axialen Längsachse des Permanentmagneten. Darüber hinaus befindet sich im axialen Überlappungsbereich des ersten Permanentmagneten und des zweiten Permanentmagneten das Gehäuse zwischen den beiden Permanentmagneten.

Die Pumpe weist ferner ein erstes Lager auf zur relativen axialen Positionierung des Rotors und des Gehäuses zueinander und zur Aufnahme einer Axialkraft, welche aus der Anordnung des ersten Permanentmagneten und zweiten Permanentmagneten resultiert.

Das Rotorlagerungssystem weist darüber hinaus ein zweites Lager und ein drittes Lager zur Aufnahme von Radialkräften und zur Positionierung der Rotationsachse des zweiten Permanentmagneten auf.

Durch den axialen Versatz des ersten zum zweiten Permanentmagneten relativ zueinander stellt sich zwischen diesen Körpern eine Kraft in axialer Richtung ein, die abhängig von der Art der Magnetisierung in oder bevorzugt entgegen der Versatzrichtung wirkt. Insbesondere lässt sich dadurch eine aus der Kupplung und anderen Betriebskräften, z. B. aus Strömungskräften, resultierende positive, negative oder verschwindende Axialkraft definiert einstellen, ohne gleichzeitig das übertragbare Drehmoment maßgeblich zu reduzieren. Dazu kann zunächst die aus der Strömung auf den Rotor ausgeübte Axialkraft bestimmt werden, z. B. durch Auswertung von Strömungssimulationen. Aus magnetischen Simulationen und/oder Messungen kann der Zusammenhang zwischen Axialversatz und magnetischer Axialkraft bestimmt werden. Anschließend kann für die Konstruktion ein Versatz gewählt werden, bei dem die magnetische Axialkraft die Strömungsaxialkraft mindestens kompensiert, vorzugsweise um einen Sicherheitsfaktor überkompensiert.

In Kombination mit dem ersten Lager, d. h. einem Axiallager, kann somit über den relativen axialen Versatz eine für die Lagerfunktion erforderliche, definierte Axialkraft eingestellt werden. Letztgenannte kann hierbei so gewählt werden, dass das erste Lager hinsichtlich Reibleistung und Verschleiß im zulässigen Bereich betrieben werden kann. Es ist dabei bevorzugt, dass das Axiallager dauerhaft im Kontakt betrieben wird, das heißt die Magnetaxialkraft muss zu jedem Zeitpunkt die Strömungsaxialkraft mindestens kompensieren. Im Gegensatz zu einer im Stand der Technik realisierten Lösung mit zwei axialen Festkörperlagern mit den oben beschriebenen Nachteilen enthält die hier beschriebene Lösung lediglich ein axiales Festkörperlager und vermeidet dadurch eine Über- bzw. Unterbestimmtheit. Durch die definiert einstellbare Axialkraft aus der Magnetkupplung ist zudem sichergestellt, dass die axiale Lagerung durch das Festkörperlager in nur eine Richtung ausreichend ist.

Gemäß einer bevorzugten Ausführungsform sind der erste Permanentmagnet und der zweite Permanentmagnet koaxial angeordnet. Dies ermöglicht in vorteilhafter Weise eine effiziente Kopplung des ersten und des zweiten Permanentmagneten.

Es ist weiter bevorzugt, dass eine Rotationsachse des Rotors und eine Achse des zweiten Permanentmagneten koaxial sind. Hierdurch wird vorteilhafterweise erreicht, dass der Rotor und der zweite Permanentmagnet symmetrisch angeordnet sind, was auch die Herstellung des Rotors erleichtert.

Es ist weiter bevorzugt, dass eine Rotationsachse der Welle und eine Achse des ersten Permanentmagneten koaxial sind. Hierdurch wird vorteilhafterweise erreicht, dass die Welle und der erste Permanentmagnet einfach herzustellen sind.

Es ist weiter bevorzugt, dass eine Rotationsachse der Welle und eine Rotationsachse des Rotors koaxial sind. Hierdurch wird vorteilhafterweise erreicht, dass die Kopplung zwischen dem ersten und dem Permanentmagneten effizient ist.

Gemäß einer bevorzugten Ausführungsform weist der Rotor einen konusförmigen oder kegelförmigen Teil auf, welcher sich an den hohlzylinderförmigen Teil anschließt. Hierbei ist es bevorzugt, dass die Achse des Konus und die Rotationsachse des Rotors, welche bevorzugt koaxial mit der Achse des zweiten Permanentmagneten ist, koaxial sind. Hierbei schließt sich an den hohlzylinderförmigen Teil in Richtung des zwischen dem Rotor und dem Gehäuse angebrachten Lagers die Grundfläche des konusförmigen Teils an. Hierbei ist der äußere Umfang der Grundfläche des Konus mit der ringförmigen Öffnung an einem axialen Ende des hohlzylinderförmigen Teils verbunden.

Gemäß einer weiteren bevorzugten Ausführungsform ist das zweite Lager am vom Gehäuse abgewandten Ende des Rotors, d. h. am vom Gehäuse abgewandten Ende des konusförmigen Teils angebracht. Hierbei ist es bevorzugt, dass das Lager zwischen dem Rotor und einem befestigten Bauteil angebracht ist, wobei das befestigte Bauteil bevorzugt mit dem Gehäuse fest verbunden ist.

Gemäß einer weiteren bevorzugten Ausführungsform weist der Rotor am äußeren Umfang des Rotors, bevorzugt am konusförmigen Teil des Rotors, Schaufeln auf, welche bei Drehung des Rotors eine Flüssigkeit von dem vom Gehäuse abgewandten Ende des Rotors in Richtung des Gehäuses transportiert. Hierbei ist es bevorzugt, dass der Rotor Löcher aufweist, sodass die Flüssigkeit von außerhalb des Rotors in einen Spalt, welcher durch die Innenseite des hohlzylinderförmigen Teils des Rotors und eine Außenseite des Gehäuses gebildet wird, angesogen wird, um dann aus dem Inneren des hohlzylinderförmigen Teils des Rotors durch den konusförmigen Teil des Rotors zu dem vom Gehäuse abgewandten Ende des Rotors zu strömen.

Gemäß einer bevorzugten Ausführungsform bilden das erste Lager und das dritte Lager gemeinsam ein kombiniertes axiales und radiales Lager, welches der Aufnahme von Axial- und Radialkräften dient. Bevorzugt weist das kombinierte axiale und radiale Lager ein axiales Lager und ein radiales Lager auf.

Gemäß einer bevorzugten Ausführungsform sind das erste Lager und das dritte Lager zwischen dem Gehäuse und dem Rotor und das zweite Lager am Rotor angeordnet. Bevorzugt ist hierbei das erste und dritte Lager ein kombiniertes axiales und radiales Lager. Hierbei wird der axiale Versatz des ersten und zweiten Permanentmagneten so eingestellt, dass das Gehäuse in Richtung des Rotors und/oder der Rotor in Richtung des Gehäuses gedrückt wird.

Gemäß einer bevorzugten Ausführungsform ist hierbei das kombinierte axiale und radiale Lager ein Festkörperlager, welches bevorzugt eine im Rotor angebrachten Kugel aufweist, welche in einem am Gehäuse angebrachten Konus rotiert, wodurch sowohl radiale als auch axiale Kräfte aufgenommen werden können. Bevorzugt weist die Kugel und/oder der Konus als Material monokristallinen Korund oder Saphir auf bzw. besteht aus diesem. Diese Materialien bieten sich aufgrund ihrer hohen Verschleißbeständigkeit an.

Gemäß einer bevorzugten Ausführungsform sind das erste Lager und das dritte Lager am Rotor und das zweite Lager zwischen dem Gehäuse und dem Rotor angeordnet.

Bevorzugt sind das erste und dritte Lager an einem vom Gehäuse abgewandten Ende des Rotors angebracht. Hierbei ist das erste und dritte Lager zwischen dem vom Gehäuse abgewandten Ende des Rotors und einem befestigten Bauteil angebracht, wobei das befestigte Bauteil bevorzugt mit dem Gehäuse fest verbunden ist.

Hierbei bilden das erste und dritte Lager bevorzugt ein kombiniertes axiales und radiales Lager. Gemäß einer bevorzugten Ausführungsform ist hierbei das kombinierte axiale und radiale Lager ein Festkörperlager, welches bevorzugt eine im Rotor angebrachten Kugel aufweist, welche in einem an dem befestigtem Bauteil angebrachten Konus rotiert, wodurch sowohl radiale als auch axiale Kräfte aufgenommen werden können. Bevorzugt weist die Kugel und/oder der Konus als Material monokristalliner Korund oder Saphir auf bzw. besteht aus ihnen.

Gemäß einer bevorzugten Ausführungsform sind das erste und das dritte Lager durch zwei verschiedene Konstruktionselemente realisiert. Z. B. kann die Axiallagerfunktion durch den Kontakt einer im Rotor angebrachten Kugel oder sonstigen vorzugsweise konvexen Fläche mit einer am Gehäuse befestigten vorzugsweise ebenen Platte realisiert werden. Die Radiallagerfunktion kann z. B. durch ein hydrodynamisches Gleitlager am Umfang des Rotors, bevorzugt am hohlzylinderförmigen Teil des Rotors auf Höhe des zweiten Permanentmagneten, realisiert werden.

Bevorzugt ist das zweite oder dritte Lager ein hydrodynamisches Gleitlager. Bevorzugt ist das zweite oder dritte Lager am Umfang des Rotors angeordnet.

Gemäß einer bevorzugten Ausführungsform ist das Gehäuse ein Motorgehäuse, welches im Inneren eine drehbar gelagerte Welle und den auf der Welle angeordneten ersten Permanentmagneten aufweist. Es ist weiter bevorzugt, dass in dem Motorgehäuse ein Motor angeordnet ist, welcher die Welle antreibt. Ferner ist es bevorzugt, dass der Motor durch das Motorgehäuse vollständig von der Umgebung abgekapselt ist, so dass ein Eindringen von Flüssigkeit in das Motorgehäuse und den Motor verhindert wird und weiterhin das Austreten von Stoffen aus dem Motorinneren in die umgebende Flüssigkeit vermieden wird.

Gemäß einer bevorzugten Ausführungsform weist sowohl der erste Permanentmagnet als auch der zweite Permanentmagnet jeweils mindestens ein Polpaar auf. Bevorzugt weist hierbei der erste Permanentmagnet dieselbe Polpaarzahl wie der zweite Permanentmagnet auf. Es ist ferner bevorzugt, dass die Polpaarzahl größer als zwei ist. Hierdurch kann vorteilhafterweise erreicht werden, dass das übertragbare Drehmoment erhöht werden kann.

Gemäß einer bevorzugten Ausführungsform ist die aus der Kupplung herrührende Axialkraft, d. h. eine magnetische Kraft, welche aus dem Versatz des ersten und zweiten Permanentmagneten resultiert und welche auf den Rotor wirkt, größer als die hydraulische Kraft gewählt. Hierbei wird unter der hydraulischen Kraft eine auf den Rotor wirkende, der Strömungsrichtung entgegen wirkende Reaktionskraft verstanden. Durch dieses Merkmal wird vorteilhafterweise erreicht, dass der Rotor am ersten Lager in Richtung des Gehäuses gedrückt wird, sodass der Rotor und das Gehäuse am ersten Lager den Kontakt nicht verlieren. Bevorzugt überkompensiert die magnetische Axialkraft die Strömungsaxialkraft um einen Sicherheitsfaktor.

Gemäß einer anderen bevorzugten Ausführungsform ist die aus der Kupplung herrührende Axialkraft kleiner als die hydraulische Kraft gewählt. Hierbei ist das erste Lager nur im Stillstand in Kontakt. Hierbei ist die hydraulische Kraft auf den Rotor im Nennbetriebspunkt zu verstehen. In diesem Fall ist im Betrieb die axiale Position vollständig durch das Gleichgewicht zwischen magnetischer und hydraulischer Kraft bestimmt.

Gemäß einer bevorzugten Ausführungsform weist sowohl der erste Permanentmagnet als auch der zweite Permanentmagnet jeweils mindestens zwei axiale Segmente auf.

Durch eine Teilung der Radialkupplung, welche durch den ersten und zweiten Permanentmagneten realisiert wird, in zwei oder mehr Segmente in axialer Richtung bei gleichzeitigem axialem Versatz der Segmente zueinander kann vorteilhafterweise erreicht werden, dass die Axialkraft erhöht wird. Bei vergleichbaren Maßen, Gesamtlänge und Außendurchmesser, nimmt dadurch das übertragbare Drehmoment ab, was jedoch durch eine axiale Verlängerung der Radialkupplung oder Erhöhung der Polpaarzahl kompensiert werden kann. Somit kann durch die Polpaarzahl, die äußeren Maße und die Unterteilung mit Abständen zwischen den Segmenten sowohl das Drehmoment als auch die Axialkraft eingestellt werden. Die Anzahl der Segmente und der Abstand zwischen den Segmenten bestimmt hierbei das Maß der Axialkraft.

Diese Maßnahme kann zum Beispiel ergriffen werden, wenn die Magnetaxialkraft nicht ausreicht, um die Strömungskraft zuverlässig zu kompensieren.

Bevorzugt ist die Segmentanzahl des ersten Permanentmagneten genau so groß wie die Segmentanzahl des zweiten Permanentmagneten. Dies vereinfacht die Produktion und erhöht die Symmetrie der Vorrichtung.

Bevorzugt weist der erste Permanentmagnet dieselbe axiale Gesamtlänge wie der zweite Permanentmagnet auf. Hierbei wird unter der axialen Gesamtlänge die Summe aller Segmente und aller Distanzstücke verstanden. Hierbei wird angenommen, dass zwischen einem Segment und einem Distanzstück oder einem anderen Segment keine Lücke vorhanden ist.

Gemäß einer bevorzugten Ausführungsform ist zwischen benachbarten Segmenten des ersten Permanentmagnets und/oder des zweiten Permanentmagnets jeweils ein Distanzstück angeordnet. Hierdurch kann vorteilhafterweise erreicht werden, dass die beiden benachbarten Segmente eines Permanentmagneten einen vorgegebenen axialen Abstand zueinander haben. Ferner kann dadurch eine axiale Vorspannung realisiert werden, z. B. um eine definierte Axialkraft für eine Lagerfunktion herstellen zu können.

Gemäß einer bevorzugten Ausführungsform weist mindestens ein Distanzstück Kunststoff, Aluminium, Titan oder ein anderes nichtmagnetisches Material auf oder besteht daraus. Dies hat den Vorteil, dass das Material des Distanzstücks das Magnetfeld nicht oder nur sehr wenig beeinflusst, da das genannte Material nicht ferromagnetisch ist.

Gemäß einer bevorzugten Ausführungsform weist der zweite Permanentmagnet eine Vorrichtung zum magnetischen Rückschluss auf. Hierbei ist diese Vorrichtung bevorzugt auf der Außenseite des zweiten Permanentmagneten angeordnet. Neben fertigungstechnischen Vorteilen wird dadurch vorteilhafterweise erreicht, dass das Drehmoment der Kupplung erhöht wird, da weniger Streufelder verloren gehen.

Gemäß einer bevorzugten Ausführungsform weist der erste Permanentmagnet und/oder der zweite Permanentmagnet eine radiale, parallele oder diametrale Magnetisierung auf. Dies sind übliche Magnetisierungsarten, welche der Fachmann an die gegebenen Umstände des Einzelfalls anpassen kann.

Gemäß einer bevorzugten Ausführungsform weist der erste Permanentmagnet und/oder der zweite Permanentmagnet einen Permanentmagneten auf, welcher eine Halbach-Anordnung aufweist oder ist, d.h. der insbesondere die Magnet-Konfiguration einer Halbach-Anordnung hat.

Unter einem Permanentmagnet, der die Magnet-Konfiguration einer Halbach-Anordnung hat, wird vorliegend ein Permanentmagnet verstanden, bei dem sich die magnetische Flussdichte auf einer Seite, der sogenannten schwachen Seite gering ist, weil der magnetische Fluss dort im Wesentlichen aufgehoben ist, und auf einer anderen Seite, der sogenannten starken Seite groß ist, weil der magnetische Fluss dort verstärkt ist.

Unter einer Halbach Anordnung werden vorliegend Anordnungen von Magneten verstanden, wie sie unter dem Link https://en.wikipedia.org/wiki/Halbach_array beschrieben sind, worauf hiermit Bezug genommen und dessen Offenbarung in die Beschreibung dieser Erfindung vollumfänglich mit einbezogen wird.

Die Magnet-Konfiguration einer Halbach-Anordnung kann durch permanentmagnetische Segmente gebildet werden, die zusammengesetzt sind und deren Magnetisierungsrichtung gegeneinander jeweils um 90° in Bezug auf eine Vorzugsrichtung, z.B. in Bezug auf die Richtung einer Längsachse der Anordnung gekippt ist. Auf diese Weise lässt sich eine seitenabhängige Flussverstärkung erzielen. Im Übrigen sei auch auf die einschlägige Fachliteratur betreffend Halbach-Anordnungen verwiesen.

Bevorzugt ist oder sind der erste Permanentmagnet und/oder der zweite Permanentmagnet ein Permanentmagnet, welcher eine Halbach-Anordnung aufweist oder ist. Durch dieses Merkmal wird vorteilhafterweise erreicht, dass der magnetische Fluss auf einer Seite der Halbach-Anordnung konzentriert werden kann (starke Seite). Dies ist besonders bei dem zweiten Permanentmagneten, welcher außenliegend angeordnet ist, vorteilhaft, wobei die starke Seite der Halbach-Anordnung des zweiten Pannenmagneten zum ersten Permanentmagneten gerichtet ist.

Der erste Permanentmagnet und der zweite Permanentmagnet sind dermaßen magnetisiert, dass bei Rotation des ersten Permanentmagneten der zweite Permanentmagnet in Rotation versetzt wird und umgekehrt. Diese Eigenschaft wird benötigt, um ein Drehmoment von dem einen Permanentmagneten berührungslos auf den anderen Permanentmagneten übertragen zu können.

Bevorzugt bilden der erste Permanentmagnet und der zweite Permanentmagnet zusammen eine magnetische Kupplung, die aufgrund der bevorzugt radial gerichteten Magnetfeldlinien bevorzugt eine Radialmagnetkupplung ist.

Gemäß einer bevorzugten Ausführungsform ist durch Variation von mindestens einem Parameter aus der nachfolgenden Liste eine auf den Rotor in der Pumpe wirkende Axialkraft des Rotorlagerungssystems frei einstellbar. Hierbei weist die Liste auf: eine Polpaarzahl des ersten Permanentmagneten und des zweiten Permanentmagneten, die Maße der Segmente des ersten Permanentmagneten, die Maße der Segmente des zweiten Permanentmagneten, Abstände zwischen benachbarten Segmenten des ersten Permanentmagneten und des zweiten Permanentmagneten, Abstände zwischen benachbarten Segmenten des magnetischen Rückschlusses, axiale Längen von Distanzstücken zwischen Segmenten des ersten Permanentmagneten und des zweiten Permanentmagneten und Segmenten des magnetischen Rückschlusses, eine Magnetisierung des ersten Permanentmagneten, eine Magnetisierung des zweiten Permanentmagneten, eine Strömungskraft, welche bei bestimmungsgemäßem Gebrauch auf den Rotor wirkt und einen Versatz des ersten Permanentmagneten gegenüber dem zweiten Permanentmagneten.

Der Fachmann versteht, dass die in der Liste genannten Variablen die Axialkraft beeinflussen. Durch Variation mindestens eines der Werte der Liste, bevorzugt mehrerer Werte der Liste, kann die Axialkraft innerhalb vorgegebener Grenzen frei eingestellt werden. Hierdurch kann vorteilhafterweise erreicht werden, dass die Axialkraft an die gegebenen Umstände des Einzelfalls geeignet angepasst werden kann.

Bevorzugt weist der Rotor mindestens eine Bohrung oder mindestens ein Loch, bevorzugt Bohrungen oder Löcher auf. Hierdurch wird vorteilhafterweise erreicht, dass die vom Rotor transportierte Flüssigkeit in einen Zwischenraum oder Spalt zwischen dem Rotor und dem Gehäuse strömen kann. Diese ermöglicht es, dass Wärme abgeführt werden kann, welche z. B. durch Reibung oder durch Wirbelströme in einem möglichen Metallgehäuse erzeugt wird. Weiterhin verhindert der kontinuierliche Fluss des Mediums idealerweise Ablagerungen von Festkörperteilen des Mediums im Bereich des Spaltes und des Lagers.

Vorteilhafte Ausführungsbeispiele der Erfindung sind in den Zeichnungen dargestellt und werden in der nachfolgenden Beschreibung näher erläutert.
- Fig. 1: zeigt eine Pumpe für das Fördern eines Blutstroms zur Herz-Kreislauf-Unterstützung gemäß einer Ausführungsform der Erfindung.
- Fig. 2: zeigt eine Schnittansicht durch eine erfindungsgemäße Ausführungsform der Pumpe an einer Stelle, wo sich der erste Permanentmagnet, welcher im Gehäuse angebracht ist, und der zweite Permanentmagnet, welcher im Rotor angeordnet ist, überlappen.
- Fig. 3 und 4: zeigen jeweils eine Pumpe für das Fördern eines Blutstroms zur Herz-Kreislauf-Unterstützung gemäß weiteren Ausführungsformen der Erfindung.

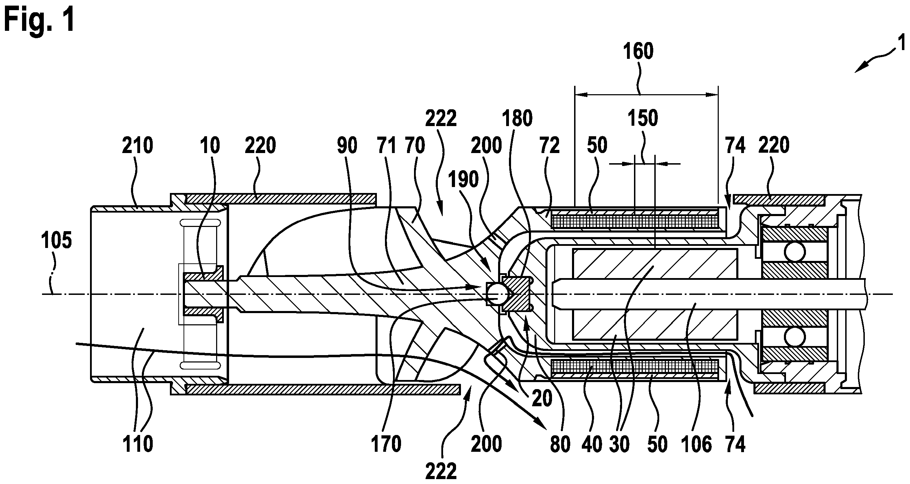

Fig. 1 zeigt eine Pumpe für das Fördern eines Blutstroms zur Herz-Kreislauf-Unterstützung (VAD) 1 mit einer berührungsfreien Drehmomentübertragung und einer radialen und axialen Lagerung eines Rotors.

Die Pumpe 1 weist ein Gehäuse 80, hier ein Motorgehäuse, auf, in dem ein erster zylinderförmiger Permanentmagnet 30 auf einer von einem nicht dargestellten Motor angetriebene Welle 106 sitzend um eine erste Achse 105 drehbar gelagert ist. Das Gehäuse 80 hat einen Außendurchmesser von 3,5 mm.

Die Pumpe 1 weist ferner einen Rotor 70 zur Förderung einer Flüssigkeit auf, wobei der Rotor 70 einen zweiten hohlzylinderförmigen Permanentmagneten 40 aufweist, welcher ebenfalls um die erste Achse 105 drehbar gelagert ist. Der zweite hohlzylinderförmige Permanentmagnet 40 ist in einem hohlzylinderförmigen Teil 72 des Rotors 70 angebracht.

Der zweite hohlzylinderförmige Permanentmagnet 40 weist an seiner Außenseite einen magnetischen Rückschluss 50 auf.

Der erste Permanentmagnet 30 hat einen Außendurchmesser von 3 mm, eine Magnethöhe von 1 mm und eine Länge von 5 mm. Der zweite Permanentmagnet 40 hat einen Außendurchmesser von 5 mm, eine Magnethöhe von 0,5 mm und eine Länge von 5 mm. Der Rotor 70 hat einen Außendurchmesser von 5,3 mm, eine Länge von 15 mm.

Der Rotor 70 ist als Laufrad ausgeführt, welcher die durch die Kupplung übertragene mechanische Leistung in hydraulische Leistung zur Förderung eines Blutstroms gegen einen Blutdruck wandelt.

Der Rotor 70 weist ferner einen konusförmigen oder kegelförmigen Teil 71 auf, welcher sich an den hohlzylinderförmigen Teil 72 anschließt. Der äußere Umfang der Grundfläche des konusförmigen Teils 71 ist mit der ringförmigen Öffnung an einem axialen Ende des hohlzylinderförmigen Teils 72 verbunden.

Der erste Permanentmagnet 30 und der zweite Permanentmagnet 40 überlappen sich zumindest teilweise axial im durch Bezugszeichen 160 gekennzeichneten axialen Bereich.

Hierbei ist der erste Permanentmagnet 30 gegenüber dem zweiten Permanentmagneten 40 axial versetzt angeordnet. Die Mitten des ersten Permanentmagneten 30 und des zweiten Permanentmagneten 40 sind durch vertikale gestrichelte Linien markiert, wobei der axiale Versatz 150 zwischen diesen beiden vertikal gestrichelten Linien eingezeichnet ist.

Aufgrund des axialen Versatzes 150 erfährt der zweite Permanentmagnet 40 eine in Fig. 1 nach rechts gerichtete Kraft, so dass eine Kugel 170, welche im Rotor 70 angebracht ist, auf einen im Gehäuse 80 angebrachten Konus 180 gedrückt wird, so dass ein erstes Lager 20 und ein drittes Lager 90, welche hier ein kombiniertes axiales und radiales Lager 190 bilden, in Kontakt gehalten wird. Bei bestimmungsgemäßem Gebrauch rotiert die Kugel 170 in dem Konus 180, wodurch sowohl radiale als auch axiale Kräfte aufgenommen werden können. Das kombinierte axiale und radiale Lager 190 ist hier ein Festkörperlager. Die Kugel 170 ist in dem konusförmigen Teil 71 angebracht. Die axiale und radiale Lagerfunktion wird durch die Kombination der beiden Elemente Kugel 170 und Konus 180 erreicht.

Die Kugel 170 hat einen Durchmesser von 0,5 mm. Der Konus 180 hat einen Durchmesser von 1 mm, eine Höhe von 0,8 mm und einen Konuswinkel von 90°.

Die axiale Lagerfunktion des kombinierten Lagers 190 hat die Funktion des ersten Lagers und dient der relativen axialen Positionierung des Rotors 70 und des Gehäuses 80 bzw. der Welle 106 zueinander sowie der Aufnahme einer Axialkraft, welche aus der Anordnung des ersten Permanentmagneten 30 und des zweiten Permanentmagneten 40 resultiert.

Ferner kann bei der Pumpe 1 die Axialkraft frei eingestellt werden, wodurch die angreifenden Kräfte optimal eingestellt werden können.

Das Gehäuse 80, welches den ersten Permanentmagneten 30 aufweist, ist im Überlappungsbereich 160 sowie in dem Bereich zwischen dem Überlappungsbereich 160 und dem Rotor 70 vom Rotor 70, insbesondere vom Inneren des hohlzylinderförmigen Teils 72 des Rotors 70 umgeben. Zwischen dem Gehäuse 80 und dem Rotor 70 bildet sich somit ein hohlzylinderförmiger Kanal 74, durch den die Flüssigkeit strömen kann. Damit Flüssigkeit von außerhalb des konusförmigen Teils 71 des Rotors 70 in den Kanal 74 kontinuierlich strömen kann, bringt man Bohrungen 200 in dem Rotor 70 an, bevorzugt in dem konusförmigen Teil 71 des Rotors 70, bzw. an einem Übergang des konusförmigen Teils 71 zum hohlzylinderförmigen Teil 72 des Rotors 70. Hierbei deuten Pfeile 110 die Fließrichtung der Flüssigkeit an.

Ein zweites Lager 10, welches als radiales, hydrodynamisches und blutgeschmiertes Gleitlager ausgeführt ist, ist am vom Gehäuse 80 abgewandten Ende des konusförmigen Teils 71 des Rotors 70 angebracht. Das zweite Lager 10 dient der Aufnahme von Radialkräften und zur Positionierung der Rotationsachse des zweiten Permanentmagneten 40, welcher in dem Rotor 70 angebracht ist. Hierbei ist das zweite Lager 10 zwischen dem Rotor 70 und einem Einsatz 210 angebracht, welches an einem zweiten Gehäuse 220 in einem ringförmigen Ende befestigt, insbesondere eingeklemmt oder eingepresst, ist, welches wiederum am Gehäuse 80 befestigt ist. Hierbei bildet das zweite Gehäuse 220 eine äußere Haut der Pumpe 1, wobei in dem zweiten Gehäuse 220, welches auch Laufradgehäuse genannt werden kann, zahlreiche Ausnehmungen 222 vorhanden sind. Bevorzugt ist der Einsatz 210 ein Lagerstern, welcher in dem zweiten Gehäuse 220 geklebt, verschweißt oder eingepresst werden kann. Der Lagerstern 210 hat einen Außendurchmesser von 6 mm und eine Länge von 3 mm. Das zweite Gehäuse 220 hat einen Außendurchmesser von 6 mm, eine Länge von 18 mm und eine Wandstärke von 0,25 mm.

Das Lager 10 hat einen Durchmesser von 1 mm und eine Länge von 1 mm.

Durch den durch die Konstruktion festgelegten axialen Versatz 150 zwischen den ersten Permanentmagneten 30 und den zweiten Permanentmagneten 40 wirkt im Ausführungsbeispiel der Fig. 1 eine definierte axiale Kraft auf den Rotor 70 in Richtung des Motors, d. h. von links nach rechts im Ausführungsbeispiel der Fig. 1. Dieser Kraft entgegen wirkt eine hydraulische Kraft auf den Rotor 70, d. h. von rechts nach links im Ausführungsbeispiel der Fig. 1. Vorliegend ist die aus der Kupplung des ersten Permanentmagneten 30 und des zweiten Permanentmagneten 40 herrührende Axialkraft leicht größer als die hydraulische Kraft gewählt. Hierdurch wird einerseits sichergestellt, dass der Rotor 70 stets definiert axial positioniert ist und andererseits das kombinierte axiale und radiale Lager 190 nicht unnötig belastet wird, wodurch Reibung und Verschleiß niedrig gehalten werden. Zur Optimierung des Reib- und Verschleißverhaltens kann außerdem der Konuswinkel des Konus 180 vergrößert werden, wobei eine ausreichende radiale Tragfähigkeit sicherzustellen ist.

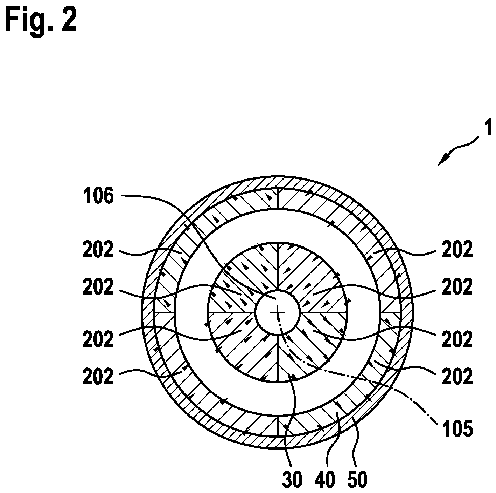

Fig. 2 zeigt eine Schnittansicht der Pumpe 1 an einer Stelle, wo sich der erste Permanentmagnet 30, welcher im Gehäuse 80 angebracht ist, und der zweite Permanentmagnet 40, welcher im hohlzylinderförmigen Teil 72 des Rotors 70 angeordnet ist, axial überlappen. Man erkennt, dass der erste Permanentmagnet 30 auf der vom Motor angetriebenen Welle 106 sitzt, welche um die erste Achse 105 drehbar gelagert ist. Ferner erkennt man, dass der zweite Permanentmagnet 40 ebenfalls um die erste Achse 105 drehbar gelagert ist. Sowohl der erste Permanentmagnet 30 als auch der zweite Permanentmagnet 40 weisen jeweils zwei Polpaare, d. h. jeweils vier Pole 202, auf, welche jeweils radial magnetisiert sind, was durch kleine Pfeile angedeutet ist.

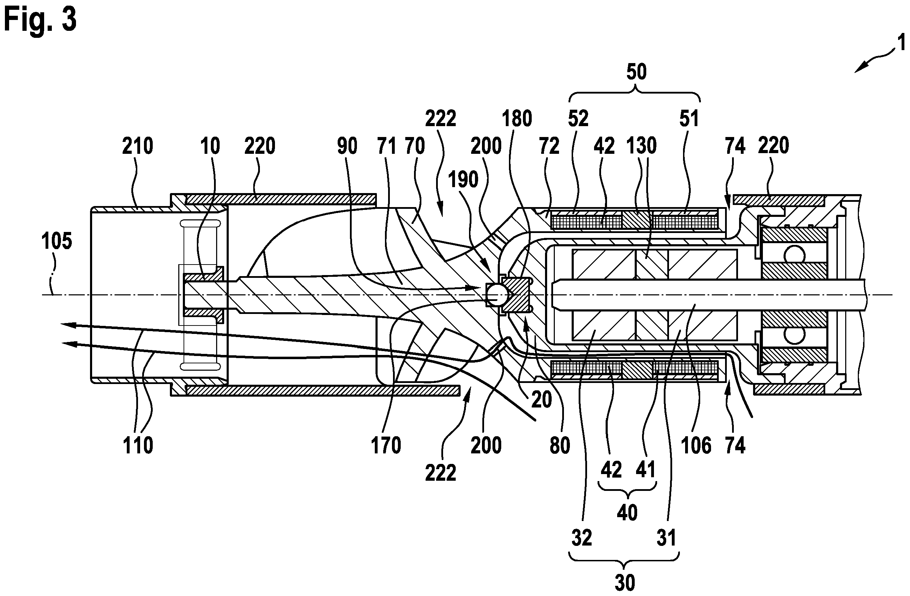

Fig. 3 zeigt eine Pumpe 1 in einer ähnlichen Ausführungsform wie die Pumpe 1 der Fig. 1. Die vorliegende Ausführungsform unterscheidet sich von der Ausführungsform der Fig. 1 darin, dass der erste Permanentmagnet 30, der zweite Permanentmagnet 40 und der magnetische Rückschluss 50 jeweils in zwei axiale Segmente aufgeteilt ist.

Der erste Permanentmagnet 30 weist die Segmente 31 und 32, der zweite Permanentmagnet 40 die Segmente 41 und 42 und der magnetische Rückschluss 50 die Segmente 51 und 52 auf. Hierbei sind die Segmente 31,41 und 51 auf der Motorseite und die Segmente 32, 42 und 52 auf der dem Rotor 70 zugewandten Seite angeordnet.

Zwischen den Segmenten 31 und 32 ist ein hohlzylinderförmiges und nicht magnetisches Distanzstück 130 angeordnet, welches ebenfalls auf der Welle 106 befestigt ist. Ein weiteres hohlzylinderförmiges und nicht magnetisches Distanzstück 130 ist zwischen den Segmenten 41 und 51 einerseits und den Segmenten 42 und 52 andererseits angeordnet.

Die Segmentierung in Kombination mit dem Versatz 150 zwischen den beiden Axialhälften führt zu einer Steigerung der Magnetaxialkraft bei gleichzeitiger Abnahme des übertragbaren Drehmoments. Diese Maßnahme wird vorliegend ergriffen, da die Magnetaxialkraft nicht ausreicht, um die Strömungskraft zuverlässig zu kompensieren.

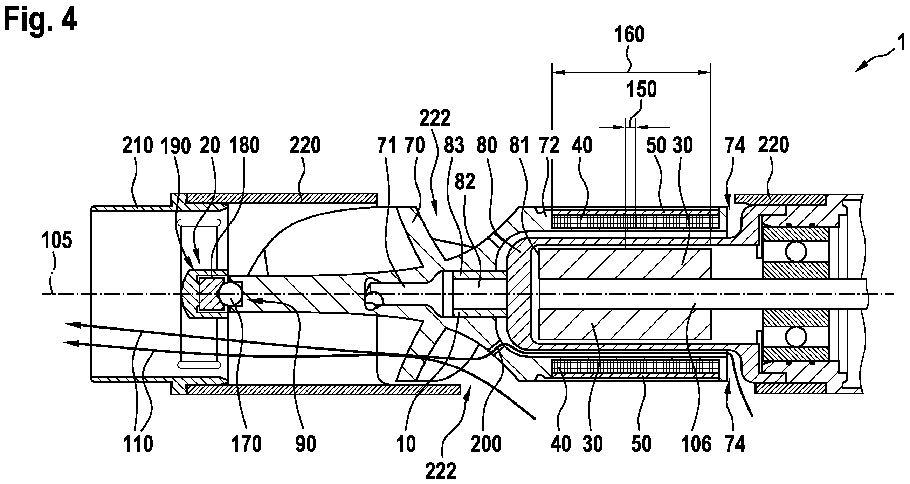

Fig. 4 zeigt eine Pumpe 1 in einer ähnlichen Ausführungsform wie die Pumpe 1 der Fig. 1 und 3. Die vorliegende Ausführungsform unterscheidet sich von der Ausführungsform der Fig. 1 darin, dass zum einem die Position des zweiten Lagers 10 mit der Position des ersten Lagers 20 und des dritten Lagers 90 vertauscht sind und zum anderen, dass der axiale Versatz 150 zwischen dem ersten Permanentmagneten 30 und den zweiten Permanentmagneten 40 in die entgegengesetzt andere Richtung zeigt wie in der Ausführungsform der Fig. 1. Der axiale Versatz 150 beträgt 1 mm in der Ausführungsform der Fig. 4.

Der erste Permanentmagnet 30 und der zweite Permanentmagnet 40 überlappen sich zumindest teilweise axial im durch Bezugszeichen 160 gekennzeichneten axialen Bereich. Hierbei ist der erste Permanentmagnet 30 gegenüber dem zweiten Permanentmagneten 40 axial versetzt angeordnet. Die Mitten des ersten Permanentmagneten 30 und des zweiten Permanentmagneten 40 sind durch vertikale gestrichelte Linien markiert, wobei der axiale Versatz 150 zwischen diesen beiden vertikal gestrichelten Linien eingezeichnet ist. Im Gegensatz zur Ausführungsform der Fig. 1 ist der erste Permanentmagnet 30 gegenüber dem zweiten Permanentmagneten 40 vom Gehäuse 80 aus gesehen in Richtung des Rotors 70 axial versetzt. Somit wirkt zwischen den ersten Permanentmagneten 30 und den zweiten Permanentmagneten 40 im Ausführungsbeispiel der Fig. 4 eine definierte axiale Kraft auf den Rotor 70, welche vom Gehäuse 80 in Richtung des Rotors 70 entlang der Achse 105 gerichtet ist, d. h. von rechts nach links im Ausführungsbeispiel der Fig. 4. In dieselbe Richtung wirkt eine hydraulische Kraft auf den Rotor 70, d. h. ebenfalls von rechts nach links im Ausführungsbeispiel der Fig. 4.

Der Vorteil dieser Anordnung ist, dass sowohl die magnetische als auch die hydraulische Axialkraft auf den Rotor 70 in dieselbe Richtung zeigen, nämlich stromaufwärts, wodurch der Rotor 70 stets in das kombinierte axiale und radiale Lager 190 gepresst wird.

Das erste Lager 20 und das dritte Lager 90 bilden hier ebenfalls ein kombiniertes axiales und radiales Lager 190, welches am vom Gehäuse 80 abgewandten Ende des konusförmigen Teils 71 des Rotors 70 angebracht ist. Hierbei ist das kombinierte Lager 190 zwischen dem Rotor 70 und einem Einsatz 210 angebracht, welches an einem zweiten Gehäuse 220 in einem ringförmigen Ende befestigt, insbesondere eingeklemmt, ist, welches wiederum am Gehäuse 80 befestigt ist. Hierbei wird eine Kugel 170, welche am vom gehäuseabgewandten Ende des konusförmigen Teils 71 des Rotors 70 angebracht ist, auf einen am Einsatz 210 angebrachten Konus 180 gedrückt.

Das zweite Lager 10, welches als radiales, hydrodynamisches Gleitlager ausgeführt ist, dient der Aufnahme von Radialkräften und zur Positionierung der Rotationsachse des zweiten Permanentmagneten 40, welcher in dem Rotor 70 angebracht ist. Das zweite Lager 10 ist hierbei zwischen dem Gehäuse 80 und dem Rotor 70 angeordnet. Im Gegensatz zur Ausführungsform der Fig. 1 weist das Gehäuse 80 der Ausführungsform der Fig. 4 hinter einer zum Rotor 70 hin gewandten Wandung 81 einen zylinderförmigen Zapfen 82 auf, welcher sich in Verlängerung der Welle 106 in Richtung des Rotors 70 fortsetzt. Der Zapfen 82 wird von einer Lagerschale 83 des das zweite Lager 10 bildenden radialen Gleitlagers umgeben.

## Patentansprüche

1. Pumpe für das Fördern eines Blutstroms zur Herz-Kreislauf-Unterstützung (1):
mit einem Rotor (70) mit einer Rotationsachse zur Förderung einer Flüssigkeit, wobei der Rotor (70) einen zweiten hohlzylinderförmigen Permanentmagneten (40) aufweist, welcher um eine Drehachse (105) drehbar gelagert ist; und
mit einem Gehäuse (80), in dem ein erster Permanentmagnet (30) zur Übertragung eines Drehmoments auf den Rotor (70) um die Drehachse (105) drehbar gelagert ist,
wobei der erste Permanentmagnet (30) und der zweite Permanentmagnet (40) sich zumindest teilweise axial überlappen, und
wobei sich in dem axialen Überlappungsbereich (160) des ersten Permanentmagneten (30) und des zweiten Permanentmagneten (40) das Gehäuse (80) zwischen den beiden Permanentmagneten (30, 40) befindet,
mit einem ersten Lager (20) zur relativen axialen Positionierung des Rotors (70) und des Gehäuses (80) zueinander und mit einem zweiten Lager (10) und einem dritten Lager (90) zur Aufnahme von Radialkräften und zur Positionierung der Rotationsachse des zweiten Permanentmagneten (40),
**dadurch gekennzeichnet, dass**
der erste Permanentmagnet (30) gegenüber dem zweiten Permanentmagneten (40) 2. axial versetzt angeordnet ist, um den Rotor (70) in dem ersten Lager (20) mit einer definierten, in der Richtung von dessen Rotorachse wirkenden resultierenden Kraft zu halten, die von einer durch die axial versetzte Anordnung des ersten Permanentmagneten (30) und des zweiten Permanentmagneten (40) hervorgerufenen Axialkraft und einer in der Richtung der Rotationsachse des Rotors (70) wirkenden hydraulischen Kraft beim Fördern des Blutstroms hervorgerufen ist.

2. Pumpe nach Anspruch 1, **dadurch gekennzeichnet, dass** das erste Lager (20) und das dritte Lager (90) gemeinsam ein kombiniertes axiales und radiales Lager (190) bilden, welches der Aufnahme von Axial- und Radialkräften dient.

3. Pumpe nach einem der vorangegangenen Ansprüche, **dadurch gekennzeichnet, dass** das erste Lager (20) und das dritte Lager (90) zwischen dem Gehäuse (80) und dem Rotor (70) und das erste zweite Lager (10) an dem Rotor (70) angeordnet sind.

4. Pumpe nach Anspruch 1 oder 2, **dadurch gekennzeichnet, dass** das erste Lager (20) und das dritte Lager (90) am Rotor (70) und das zweite Lager (10) zwischen dem Gehäuse (80) und dem Rotor (70) angeordnet ist.

5. Pumpe nach einem der vorangegangenen Ansprüche, **dadurch gekennzeichnet, dass** das Gehäuse (80) ein Motorgehäuse ist, welches im Inneren eine drehbar gelagerte Welle (106) und den auf der Welle (106) angeordneten ersten Permanentmagneten (30) aufweist.

6. Pumpe nach einem der vorangegangenen Ansprüche, **dadurch gekennzeichnet, dass** der erste Permanentmagnet (30) und der zweite Permanentmagnet (40) koaxial angeordnet sind.

7. Pumpe nach einem der vorangegangenen Ansprüche, **dadurch gekennzeichnet, dass** sowohl der erste Permanentmagnet (30) als auch der zweite Permanentmagnet (40) mindestens ein Polpaar aufweist, wobei der erste Permanentmagnet (30) dieselbe Polpaarzahl wie der zweite Permanentmagnet (40) aufweist.

8. Pumpe nach einem der vorangegangenen Ansprüche, **dadurch gekennzeichnet, dass** die durch die axial versetzte Anordnung des ersten Permanentmagneten (30) und des zweiten Permanentmagneten (40) hervorgerufene Axialkraft größer ist als die auf den Rotor (70) in der Richtung seiner Rotationsachse wirkende hydraulische Kraft beim Fördern des Blutstroms.

9. Pumpe nach einem der vorangegangenen Ansprüche, **dadurch gekennzeichnet, dass** sowohl der erste Permanentmagnet (30) als auch der zweite Permanentmagnet (40) jeweils mindestens zwei axiale Segmente (31, 32, 41, 42) aufweist.

10. Pumpe nach einem der vorangegangenen Ansprüche, **dadurch gekennzeichnet, dass** der erste Permanentmagnet (30) und/oder der zweite Permanentmagnet (40) eine radiale, parallele oder diametrale Magnetisierung aufweist.

11. Pumpe nach einem der Ansprüche 1 bis 9, **dadurch gekennzeichnet, dass** der erste Permanentmagnet (30) und/oder der zweite Permanentmagnet (40) einen Permanentmagneten aufweist, welcher eine Halbach-Anordnung aufweist oder ist.

12. Pumpe nach einem der vorangegangenen Ansprüche, **dadurch gekennzeichnet, dass** die Axialkraft durch Variation von mindestens einem Parameter aus der Liste, welche eine Polpaarzahl des ersten Permanentmagneten (30) und des zweiten Permanentmagneten (40), die Maße der Segmente (31, 32) des ersten Permanentmagneten (30), die Maße der Segmente (41, 42) des zweiten Permanentmagneten (40), Abstände zwischen benachbarten Segmenten (31, 32, 41, 42) des ersten Permanentmagneten (102) und des zweiten Permanentmagneten (104), Abstände zwischen benachbarten Segmenten (51, 52) des magnetischen Rückschlusses (50), axiale Längen von Distanzstücken (130) zwischen Segmenten (31, 32, 41, 42) des ersten Permanentmagneten (102) und des zweiten Permanentmagneten (104) und Segmenten (51, 52) des magnetischen Rückschlusses (50), eine Magnetisierung des ersten Permanentmagneten (30), eine Magnetisierung des zweiten Permanentmagneten (40), eine Strömungskraft, welche bei bestimmungsgemäßem Gebrauch auf den Rotor wirkt, und einen Versatz (150) des ersten Permanentmagneten (30) gegenüber dem zweiten Permanentmagneten (40) aufweist, frei einstellbar ist.

## Claims

1. Pump for conveying a blood flow for cardiovascular support (1):
comprising a rotor (70) having an axis of rotation for conveying a liquid, the rotor (70) having a second hollow-cylindrical permanent magnet (40) which is rotatably mounted about an axis of rotation (105); and
comprising a housing (80) in which a first permanent magnet (30) for transmitting a torque to the rotor (70) is rotatably mounted about the axis of rotation (105),
the first permanent magnet (30) and the second permanent magnet (40) at least partially axially overlapping, and
the housing (80) being located between the two permanent magnets (30, 40) in the axial overlapping region (160) of the first permanent magnet (30) and the second permanent magnet (40),
comprising a first bearing (20) for axially positioning the rotor (70) and the housing (80) relative to one another and comprising a second bearing (10) and a third bearing (90) for absorbing radial forces and for positioning the axis of rotation of the second permanent magnet (40),
**characterized in that**
the first permanent magnet (30) is arranged axially offset with respect to the second permanent magnet (40) in order to hold the rotor (70) in the first bearing (20) with a defined resulting force acting in the direction of the rotor axis of the rotor, which force is caused by an axial force caused by the axial offset arrangement of the first permanent magnet (30) and the second permanent magnet (40) and a hydraulic force acting in the direction of the axis of rotation of the rotor (70) when the blood flow is conveyed.

2. Pump according to claim 1, **characterized in that** the first bearing (20) and the third bearing (90) together form a combined axial and radial bearing (190) which is used to absorb axial and radial forces.

3. Pump according to either of the preceding claims, **characterized in that** the first bearing (20) and the third bearing (90) are arranged between the housing (80) and the rotor (70) and the second bearing (10) is arranged on the rotor (70).

4. Pump according to either claim 1 or claim 2, **characterized in that** the first bearing (20) and the third bearing (90) are arranged on the rotor (70) and the second bearing (10) is arranged between the housing (80) and the rotor (70).

5. Pump according to any of the preceding claims, **characterized in that** the housing (80) is a motor housing which has a rotatably mounted shaft (106) located therein and has the first permanent magnet (30) arranged on the shaft (106).

6. Pump according to any of the preceding claims, **characterized in that** the first permanent magnet (30) and the second permanent magnet (40) are arranged coaxially.

7. Pump according to any of the preceding claims, **characterized in that** both the first permanent magnet (30) and the second permanent magnet (40) have at least one pole pair, the first permanent magnet (30) having the same number of pole pairs as the second permanent magnet (40).

8. Pump according to any of the preceding claims, **characterized in that** the axial force caused by the axially offset arrangement of the first permanent magnet (30) and the second permanent magnet (40) is greater than the hydraulic force acting on the rotor (70) in the direction of the axis of rotation thereof when the blood flow is conveyed.

9. Pump according to any of the preceding claims, **characterized in that** both the first permanent magnet (30) and the second permanent magnet (40) each have at least two axial segments (31, 32, 41, 42).

10. Pump according to any of the preceding claims, **characterized in that** the first permanent magnet (30) and/or the second permanent magnet (40) has a radial, parallel or diametrical magnetization.

11. Pump according to any of claims 1 to 9, **characterized in that** the first permanent magnet (30) and/or the second permanent magnet (40) has a permanent magnet which has or is a Halbach arrangement.

12. Pump according to any of the preceding claims, **characterized in that** the axial force can be freely adjusted by varying at least one parameter from the list, which list has a number of pole pairs of the first permanent magnet (30) and the second permanent magnet (40), the dimensions of the segments (31, 32) of the first permanent magnet (30), the dimensions of the segments (41, 42) of the second permanent magnet (40), distances between adjacent segments (31, 32, 41, 42) of the first permanent magnet (102) and the second permanent magnet (104), distances between adjacent segments (51, 52) of the magnetic yoke (50), axial lengths of spacers (130) between segments (31, 32, 41, 42) of the first permanent magnet (102) and the second permanent magnet (104) and segments (51, 52) of the magnetic yoke (50), a magnetization of the first permanent magnet (30), a magnetization of the second permanent magnet (40), a flow force which, when used as intended, acts on the rotor, and an offset (150) of the first permanent magnet (30) with respect to the second permanent magnet (40).

## Revendications

1. Pompe pour l'acheminement d'un flux sanguin destinée à l'aide cardiovasculaire (1) :
comportant un rotor (70) comportant un axe de rotation destiné à l'acheminement d'un liquide, dans laquelle le rotor (70) comporte un second aimant permanent (40) cylindrique creux, lequel est monté rotatif autour d'un axe de rotation (105) ; et
comportant un carter (80) dans lequel un premier aimant permanent (30) destiné à la transmission d'un couple au rotor (70) est monté rotatif autour de l'axe de rotation (105),
dans laquelle le premier aimant permanent (30) et le second aimant permanent (40) se chevauchent au moins partiellement axialement, et
dans laquelle le carter (80) est situé dans la zone de chevauchement axial (160) du premier aimant permanent (30) et du second aimant permanent (40) entre les deux aimants permanents (30, 40), comportant un premier palier (20) destiné au positionnement axial relatif du rotor (70) et du carter (80) l'un par rapport à l'autre et comportant un deuxième palier (10) et un troisième palier (90) destinés à l'absorption de forces radiales et au positionnement de l'axe de rotation du second aimant permanent (40),
**caractérisée en ce que**
le premier aimant permanent (30) est disposé décalé axialement par rapport au second aimant permanent (40) afin de maintenir le rotor (70) dans le premier palier (20) avec une force résultante définie et agissant dans la direction de son axe de rotor, laquelle est provoquée par une force axiale provoquée par l'agencement décalé axialement du premier aimant permanent (30) et du second aimant permanent (40) et une force hydraulique agissant dans la direction de l'axe de rotation du rotor (70) lors de l'acheminement du flux sanguin.

2. Pompe selon la revendication 1, **caractérisée en ce que** le premier palier (20) et le troisième palier (90) forment ensemble un palier axial et radial (190) combiné, lequel sert à absorber des forces axiales et radiales.

3. Pompe selon l'une quelconque des revendications précédentes, **caractérisée en ce que** le premier palier (20) et le troisième palier (90) sont disposés entre le carter (80) et le rotor (70) et le deuxième palier (10) est disposé sur le rotor (70).

4. Pompe selon la revendication 1 ou 2, **caractérisée en ce que** le premier palier (20) et le troisième palier (90) sont disposés sur le rotor (70) et le deuxième palier (10) est disposé entre le carter (80) et le rotor (70).

5. Pompe selon l'une quelconque des revendications précédentes, **caractérisée en ce que** le carter (80) est un carter de moteur, lequel comporte à l'intérieur un arbre (106) monté rotatif et le premier aimant permanent (30) disposé sur l'arbre (106).

6. Pompe selon l'une quelconque des revendications précédentes, **caractérisée en ce que** le premier aimant permanent (30) et le second aimant permanent (40) sont disposés coaxialement.

7. Pompe selon l'une quelconque des revendications précédentes, **caractérisée en ce que** le premier aimant permanent (30) et le second aimant permanent (40) comportent au moins une paire de pôles, dans laquelle le premier aimant permanent (30) comporte le même nombre de paires de pôles que le second aimant permanent (40).

8. Pompe selon l'une quelconque des revendications précédentes, **caractérisée en ce que** la force axiale provoquée par l'agencement décalé axialement du premier aimant permanent (30) et du second aimant permanent (40) est supérieure à la force hydraulique agissant sur le rotor (70) dans la direction de son axe de rotation lors de l'acheminement du flux sanguin.

9. Pompe selon l'une quelconque des revendications précédentes, **caractérisée en ce que** le premier aimant permanent (30) et le second aimant permanent (40) comportent respectivement au moins deux segments (31, 32, 41, 42) axiaux.

10. Pompe selon l'une quelconque des revendications précédentes, **caractérisée en ce que** le premier aimant permanent (30) et/ou le second aimant permanent (40) présentent une aimantation radiale, parallèle ou diamétrale.

11. Pompe selon l'une quelconque des revendications 1 à 9, **caractérisée en ce que** le premier aimant permanent (30) et/ou le second aimant permanent (40) comportent un aimant permanent, lequel comporte ou est un agencement de Halbach.

12. Pompe selon l'une quelconque des revendications précédentes, **caractérisée en ce que** la force axiale est librement par variation d'au moins un paramètre de la liste, laquelle comporte un nombre de paires de pôles du premier aimant permanent (30) et du second aimant permanent (40), les dimensions des segments (31, 32) du premier aimant permanent (30), les dimensions des segments (41, 42) du second aimant permanent (40), des distances entre des segments adjacents (31, 32, 41, 42) du premier aimant permanent (102) et du second aimant permanent (104), des distances entre des segments adjacents (51, 52) d'une culasse magnétique (50), des longueurs axiales des pièces d'écartement (130) entre des segments (31, 32, 41, 42) du premier aimant permanent (102) et du second aimant permanent (104) et des segments (51, 52) de la culasse magnétique (50), une magnétisation du premier aimant permanent (30), une magnétisation du second aimant permanent (40), une force d'écoulement, laquelle, lorsqu'elle est utilisée comme prévu, agit sur le rotor, et un décalage (150) du premier aimant permanent (30) par rapport au second aimant permanent (40).
